# EUROPEAN PATENT SPECIFICATION

(11) **EP 3 013 344 B1**
(45) Date of publication and mention of the grant of the patent: **24.10.2018**
(21) Application number: 14817395.8
(22) Date of filing: 30.06.2014
(51) Int. Cl.: A61K 31/485, A61P 25/36

(54) **OPIOID ANTAGONISTS FOR USE IN THE TREATMENT OF AN OPIOID ANALGESIC-INDUCED ARRHYTHMIA**
OPIOID-ANTAGONISTEN ZUR BEHANDLUNG VON EINER DURCH OPIOID-ANALGESIKA VERURSACHTEN ARRHYTHMIE
ANTAGONISTES DES OPIOÏDES DESTINÉES AU TRAITEMENT D'UNE ARRYTHMIE CAUSÉE PAR LES OPIOÏDES ANALGÉSIQUES

(30) Priority: 28.06.2013 US 201361840717 P; 23.12.2013 US 201361920065 P
(43) Date of publication of application: 04.05.2016
(73) Proprietor: Purdue Pharma L.P., Stamford, CT 06901 (US)
(72) Inventor: HARRIS, Stephen, Weston, CT 06883 (US)
(74) Representative: Maiwald Patent- und Rechtsanwaltsgesellschaft mbH
(86) International application number: PCT/US2014/044878
(87) International publication number: WO 2014/210596

(56) References cited:
- WO-A2-01/85150
- US-A1- 2010 119 585
- US-A1- 2010 120 812
- US-A1- 2012 178 771
- SAM HANON ET AL: "Ventricular arrhythmias in patients treated with methadone for opioid dependence", JOURNAL OF INTERVENTIONAL CARDIAC ELECTROPHYSIOLOGY, KLUWER ACADEMIC PUBLISHERS, BO, vol. 28, no. 1, 23 February 2010 (2010-02-23), pages 19-22, XP019790596, ISSN: 1572-8595
- STALLVIK MARIANNE ET AL: "Corrected QT interval during treatment with methadone and buprenorphine-Relation to doses and serum concentrations", DRUG AND ALCOHOL DEPENDENCE, ELSEVIER SCIENTIFIC PUBLISHERS, IR, vol. 129, no. 1, 16 October 2012 (2012-10-16), pages 88-93, XP028988598, ISSN: 0376-8716, DOI: 10.1016/J.DRUGALCDEP.2012.09.016
- LEE, AYS: 'Naloxone as an Antiarrhythmic Agent' ACTA CARDIOL. SIN. vol. 5, 1989, pages 301 - 306 ., XP008182642
- THANAVARO, KL ET AL.: 'Methodone-Induced Torsades De Pointes: a Twist of Fate' HEART AND LUNG vol. 40, no. 5, 2011, pages 448 - 452, XP028279588
- LIU, XL ET AL.: 'Evaluation of Anti-Arrhythmic Potency of Naltrexone in Isolated Ischaemic Rat Heart' ACTA PHARMAOLOGICA SINICA vol. 9, no. 1, 1988, pages 40 - 43, XP008182641
- SITSAPESAN, R ET AL.: 'The Effects of Drugs Interacting with Opioid Receptors on the Early Ventricular Arrhythmias Arising from Myocardial Ishaemia' BR. J. PHARMACOL. vol. 97, 1989, pages 795 - 800 ., XP055306413
- C. A. PARONIS ET AL: "Buprenorphine and Opioid Antagonism, Tolerance, and Naltrexone-Precipitated Withdrawal", JOURNAL OF PHARMACOLOGY AND EXPERIMENTAL THERAPEUTICS, vol. 336, no. 2, 1 February 2011 (2011-02-01), pages 488-495, XP055400373, US ISSN: 0022-3565, DOI: 10.1124/jpet.110.173823

## Description

The invention is directed to an opioid antagonist for use in a method of treating or preventing an arrhythmia induced by the administration of an opioid analgesic.

### BACKGROUND OF THE INVENTION

An abnormal sequence of electrical impulses in the heart is referred to as an arrhythmia. The abnormal sequences can cause the heart to beat too quickly, too slowly or erratically. This abnormality can result in inefficient pumping of the blood which can result in fatigue, dizziness, lightheadedness, syncope, shortness of breath and chest pain. In extreme cases, arrhythmia can lead to sudden cardiac arrest and failure of organs such as the brain and lungs.

Various types of arrhythmias include atrial fibrillation (irregular contraction of the upper heart chambers), bradycardia (slow heart rate), conduction disorders, premature contraction, tachycardia (fast heart rate) and ventricular fibrillation (irregular contraction of the lower chambers of the heart).

Arrhythmias can be due to a congenital condition or acquired due to damage to the heart muscle (e.g., from a heart attack). This condition can also be acquired from exposure to chemical agents such as abnormal blood and tissue concentrations of minerals (e.g., potassium, magnesium or calcium); addictive substances (e.g., alcohol and tobacco) as well as drug therapy administered for a therapeutic effect.

Treatment of clinically significant arrhythmias includes drug therapy, ES:GGE:JS
ablation (e.g., transcatheter and catheter ablation), defibrillation, and the implantation of devices such as cardioverter defibrillators and pacemakers.

There continues to be a need for compositions and methods for the treatment of both congenital and acquired arrhythmias.

Hanon and co-workers reported on "Ventricular arrhythmias in patients treated with methadone for opioid dependence" (J Interv Card Electrophysiol 28, 19-22, 2010) and considered buprenorphine a useful and effective alternative to methadone in a select group of patients, including those with documented ventricular arrhythmias on methadone. Stallvik and co-workers investigated the "Corrected QT interval during treatment with methadone and buprenorphine - Relation to doses and serum concentrations" (Drug and Alcohol Dependence 123, 88-93, 2013) and concluded that treatment with methadone in modest doses would not be associated with clinically significant QTc increases, and that buprenorphine in commonly used doses would be a suitable alternative to methadone with regard to the risk of QTc prolongation. Thanavaro and Thanavaro reported on a case of "Methadone-induced torsades de pointes: A twist of fate" (Heart & Lung 40(5), 448-453, 2011) and observed that the corrected QT interval became shorter but remained profoundly prolonged until methadone was substituted with buprenorphine.

### OBJECTS AND SUMMARY

It is an object of certain embodiments of the invention to provide a medicament for use in a method of treating or preventing an arrhythmia in a patient in need thereof.

It is an object of certain embodiments of the invention to provide a medicament for use in a method of treating or preventing a drug induced arrhythmia in a patient in need thereof.

The above objects of the present invention and others can be achieved by the present invention as defined in the claims.

In describing the present invention, the following terms are to be used as indicated below. As used herein, the singular forms "a," "an," and "the" include plural references unless the context clearly indicates otherwise. Thus, for example, reference to "an opioid antagonist" includes a single opioid as well as a mixture of two or more different opioid antagonists.

As used herein, the term "therapeutically effective" refers to the amount of drug or the rate of drug administration needed to produce a desired therapeutic result.

As used herein, the term "prophylactically effective" refers to the amount of drug or the rate of drug administration needed to produce a desired preventive result.

The term "patient" means a subject, particularly a human, who has presented a clinical manifestation of a particular symptom or symptoms suggesting the need for treatment, who is treated preventatively or prophylactically for a condition, or who has been diagnosed with a condition to be treated. The term "subject" is inclusive of the definition of the term "patient" and does not exclude individuals who are entirely normal in all respects or with respect to a particular condition.

As used here, the term "patient in need thereof" refers to a patient experiencing or susceptible to an arrhythmia.

"Pharmaceutically acceptable salts" include inorganic acid salts such as hydrochloride, hydrobromide, sulfate, phosphate; organic acid salts such as formate, acetate, trifluoroacetate, maleate, tartrate; sulfonates such as methanesulfonate, benzenesulfonate, p-toluenesulfonate; amino acid salts such as arginate, asparaginate, glutamate; metal salts such as sodium salt, potassium salt, cesium salt; alkaline earth metals such as calcium salt, magnesium salt; and organic amine salts such as triethylamine salt, pyridine salt, picoline salt, ethanolamine salt, triethanolamine salt, dicyclohexylamine salt, N,N'- dibenzylethylenediamine salt.

The term "opioid analgesic" means any material that produces an analgesic effect through modulation of an opioid receptor, whether or not approved by a government agency for that purpose. The opioid analgesic for use in the method of the present invention is selected from the group consisting of morphine, oxycodone, tramadol, buprenorphine, oxymorphone, fentanyl, codeine, hydrocodone, hydromorphone, methadone, and pharmaceutically acceptable salts thereof. The term includes all pharmaceutically active forms of the opioid analgesic, including the free base form of the agent, and all pharmaceutically acceptable salts, complexes, crystalline forms, co-crystals, hydrates, solvates, and mixtures thereof, where the form is pharmaceutically active.

The term "concurrently" means that a dose of one agent is administered prior to the end of the dosing interval of another agent. For example, a dose of an opioid antagonist with a 12-hour dosing interval would be concurrently administered with an arrhythmia inducing active agent dose administered within 12 hours of the opioid antagonist administration.

The term "opioid naive" refers to patients who are not receiving opioid analgesics on a daily basis.

The term "opioid tolerant" means patients who are chronically receiving opioid analgesics on a daily basis.

The term "first administration" means a single dose at the initiation of therapy to an individual subject, patient, or healthy subject or a subject population, patient population, or healthy subject population.

The term "steady state" means that the amount of the drug reaching the system is approximately the same as the amount of the drug leaving the system. Thus, at "steady-state", the patient's body eliminates the drug at approximately the same rate that the drug becomes available to the patient's system through absorption into the blood stream.

The term "long QT interval" as used herein refers to a QT interval (i.e., the time interval between the Q wave and the T wave of the electrocardiogram) which is longer then approximately one-half of the R to R interval (i.e., the time elapsing between two consecutive R waves in the electrocardiogram.

The term "QTcI" as used herein refers to a QT interval which takes into account the physiologic shortening of the QT interval which occurs as the heart rate increases.

The term "acquired long QT syndrome" as used herein refers to any variation from the normal sinus rhythm of the heart, where the variation is associated with a prolonged QT interval, and the variation is not congenital. For example, the syndrome can be induced by the administration of medication. The medication may either be of any sort, including antiarrhythmic medications, other cardiac medications, and even non-cardiac medications.

The term "arrhythmia" as used herein refers to any variation from the normal sinus rhythm of the heart, where the variation is most often caused by a disturbance in the conduction properties of the atria and/or ventricles. Such variations include tachy- arrhythmia, atrial fibrilation, atrial flutter, atrial tachycardia, supraventricular tachycardia, AV nodal re-entry tachycardia, or ventricular tachycardia.

### BRIEF DESCRIPTION OF THE DRAWINGS

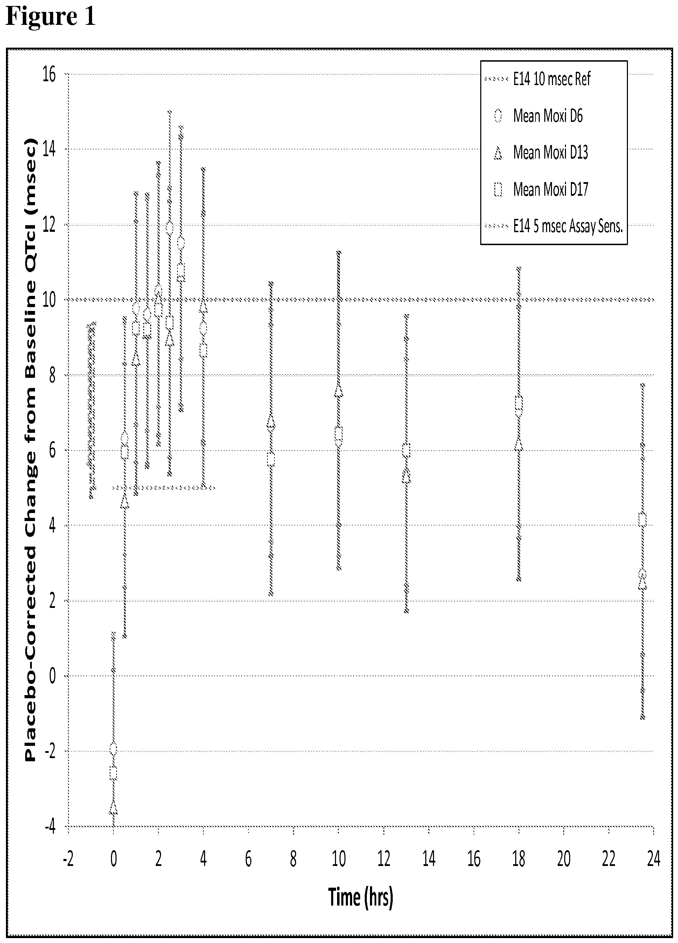

Figure 1 is a graphical depiction of the mean + 90% CI Time-Matched QTcI on Day 6, 13 and 17 for Moxifloxacin Doses (400mg) of Example 1.
Figure 2 is a graphical depiction of the mean + 90% CI Time-Matched QTcI on Day 6 for BTDS (buprenorphine transdermal delivery system) lOmcg/hr of Example 1.
Figure 3 is a graphical depiction of the mean + 90% CI Time-Matched QTcI on Day 13 for BTDS 40mcg/hr of Example 1.
Figure 4 is a graphical depiction of the mean + 90% CI Time-Matched QTcI on Day 17 for BTDS 80mcg/hr of Example 1.
Figure 5 is a graphical depiction of the mean + 90% CI Time-Matched QTcI on Day 6, 13 and 17 for BTDS 10, 40 and 80 mcg/hr of Example 1.

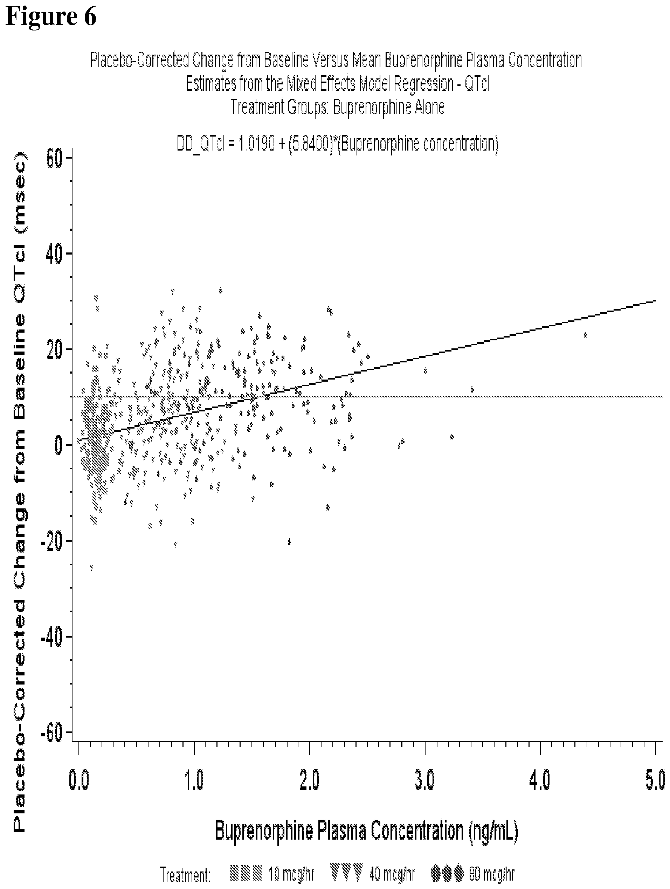

Figure 6 is a graphical depiction of the QTcI Placebo-corrected change from baseline PK/PD (pharmacokinetic/pharmacodynamic) for Example 1.

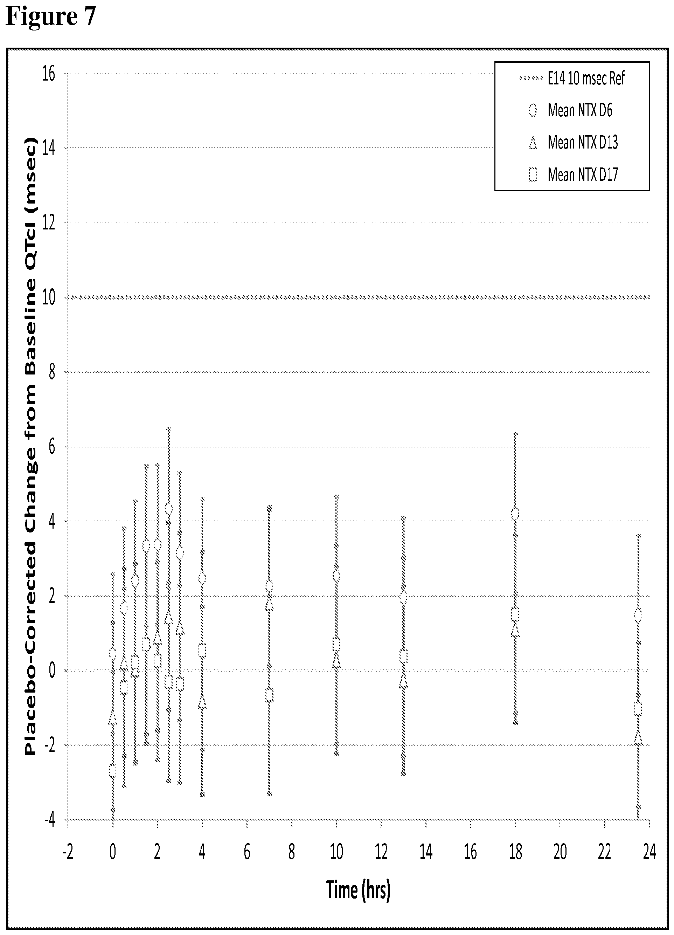

Figure 7 is a graphical depiction of the mean + 90%CI Time-Matched QTcI on Day 6, 13 and 17 for NTX (50mg ql2h) of Example 1.

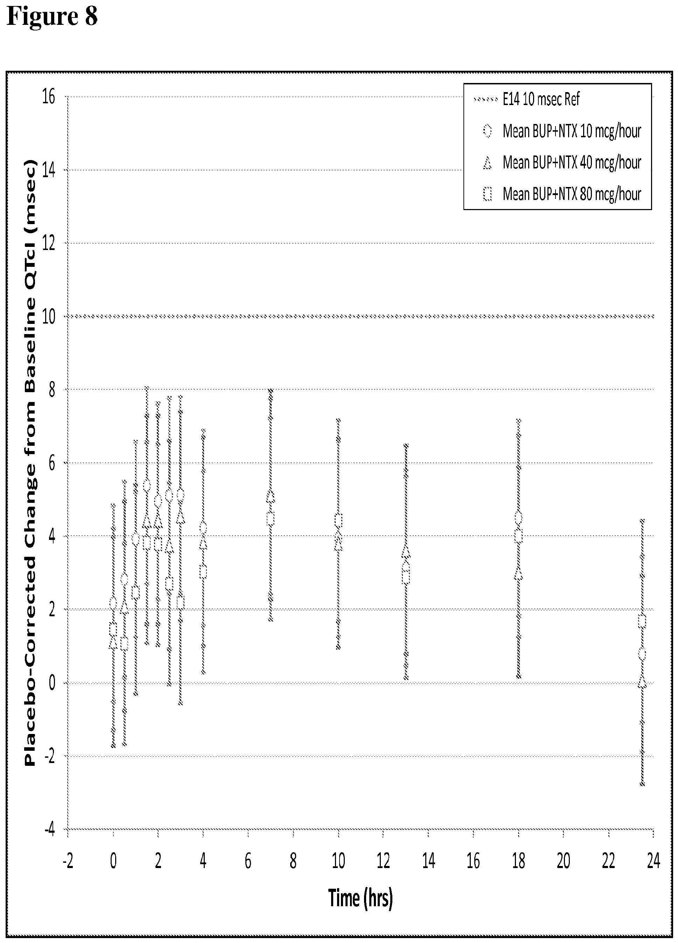

Figure 8 is a graphical depiction of the mean + 90% CI Time-Matched QTcI on Day 6, 13 and 17 for BTDS+NTX (50mg ql2h) of Example 1.

### DETAILED DESCRIPTION

The present invention relates to an opioid antagonist for use in a method of preventing or treating an arrhythmia in a patient by lowering the QTcI in said patient to less than 10 ms, wherein the arrhythmia is induced by the administration of an opioid analgesic, the method comprising administering to a patient in need thereof an effective amount of an opioid antagonist concurrently with the opioid analgesic to prevent or treat the arrhythmia, wherein the opioid analgesic is selected from the group consisting of oxycodone, morphine, codeine, oxymorphone, fentanyl, hydrocodone, hydromorphone, tramadol, buprenorphine, methadone, and pharmaceutically acceptable salts thereof, and wherein the opioid antagonist is selected from the group consisting of naltrexone, naloxone, nalmefene, cyclazacine, levallorphan, and pharmaceutically acceptable salts thereof. In a particular embodiment, the arrhythmia is a prolonged QT interval and the antagonist is naltrexone or a pharmaceutically acceptable salt thereof.

In certain embodiments, the antagonist can be administered to a patient diagnosed with a condition such as ventricular tachyarrhythmia, torsades de pointes or long QT syndrome.

The arrhythmia treated in accordance with the present invention is induced by the administration of an opioid agonist as defined herein (administered in an effective amount to provide an analgesic effect, i.e. opioid analgesic). In a particular embodiment, the opioid agonist is buprenorphine.

Buprenorphine is commonly used for its analgesic properties and is formulated, e.g., in a transdermal patch (Butrans® buprenorphine transdermal system) to provide 5 meg/hour, 10 meg/hour or 20 meg/hour of buprenorphine. Butrans® is indicated for the management of moderate to severe chronic pain in patients requiring a continuous, around-the-clock opioid analgesic for an extended period of time.

In certain patients, the transdermal administration of buprenorphine at an elevated dose may lead to a prolonged QT interval. These patients may be treated for this arrhythmia by administration of an opioid antagonist.

In certain embodiments, after treatment with an opioid antagonist in accordance with the invention, the QTcI exhibited by an individual patient is less than 10 ms, less than 8 ms or less than 6 ms.

After treatment with an opioid antagonist in accordance with the invention, the QTcI exhibited by a patient population is less than 10 ms, less than 8 ms or less than 6 ms.

In certain embodiments, after treatment with an opioid antagonist in accordance with the invention (i.e., to a patient that would otherwise exhibit a QT prolongation), the QTcI mean change from baseline exhibited by a patient population is less than 5 ms, less than 4 ms or less than 2 ms.

The arrhythmia treated by the present invention is induced by the administration of an opioid analgesic selected from the group consisting of oxycodone, morphine, codeine, oxymorphone, fentanyl, hydrocodone, hydromorphone, tramadol, buprenorphine, methadone, and pharmaceutically acceptable salts thereof.

In certain embodiments of the invention, the arrhythmia may be a side effect of buprenorphine administered transdermally in an amount greater than 20 µg/hour; from 25 µg/hour to 200 µg/hour; from 25 µg/hour to 80 µg/hour or from 40 µg/hour to 80 µg/hour.

In certain embodiments of the invention, the arrhythmia may be a side effect of buprenorphine administered transdermally with a dosing interval of 24 hours; 3 days; or 7 days.

The opioid antagonist is administered concurrently with an arrhythmia inducing opioid agonist, and the opioid antagonist serves to prevent, minimize, inhibit, ameliorate or reverse the active agent-induced arrhythmia that might otherwise occur. The opioid agonist is administered in an effective amount to provide an analgesic effect.

Preferably, the opioid antagonist is in an amount that does not decrease the analgesic effectiveness of the opioid agonist, or is in an amount that does not significantly decrease the analgesic effectiveness of the opioid agonist (i.e., there may be a slight decrease in analgesia but the patient still maintains an acceptable analgesic effect).

A patient receiving the opioid antagonist therapy of the present invention may be opioid naive. Opioid naive patients may have initiated therapy with the opioid agonist prior to initiation of the opioid antagonist therapy, or they may have initiated therapy with the opioid agonist concurrently with the initiation of the opioid antagonist therapy. In other embodiments, the opioid antagonist therapy can be initiated prior to the initiation of therapy with the opioid agonist so as to provide a prophylactic effect.

Alternatively, a patient receiving the opioid antagonist therapy of the present invention may previously have been dosed chronically with an opioid agonist so that he or she is now opioid tolerant.

The opioid antagonist therapy of the present invention can be administered after the patient begins to exhibit symptoms of an arrhythmia. Alternatively, the opioid antagonist therapy of the present invention can be administered prior to or at the same time as a patient begins treatment with the opioid agonist in order to reduce or avoid arrhythmias that might otherwise occur due to administration of the opioid agonist alone.

The opioid agonist is administered concurrently with the opioid antagonist therapy of the present invention.

The opioid antagonist administered in the present invention is selected from the group consisting of naltrexone, naloxone, nalmefene, cyclazacine, levallorphan, pharmaceutically acceptable salts, solvates, derivatives, polymorphs, and mixtures thereof.

The opioid antagonist used according to the present invention can be administered by the same route as the arrhythmia inducing active agent. For example, the opioid antagonist and the arrhythmia inducing active agent can both be administered by the same route selected from the group consisting of oral, transdermal, sublingual, buccal, intranasal, rectal, subcutaneous, intramuscular, intravenous and parenteral routes.

In alternative embodiments, the opioid antagonist used according to the present invention can be administered by a different route than the arrhythmia inducing active agent. For example, the opioid antagonist and the arrhythmia inducing active agent can be independently administered by different routes selected from the group consisting of oral, transdermal, sublingual, buccal, intranasal, rectal, subcutaneous, intramuscular, intravenous and parenteral routes.

Non-limiting examples of routes of administration for the present invention include transdermal opioid antagonist with the arrhythmia inducing active agent administered orally; transdermal opioid antagonist with the arrhythmia inducing active agent administered parenterally; transdermal opioid antagonist with the arrhythmia inducing active agent administered intranasally; transdermal opioid antagonist with the arrhythmia inducing active agent administered sublingually; and transdermal opioid antagonist with the arrhythmia inducing active agent administered transdermally.

Other routes of administration of the present invention include sublingual opioid antagonist with the arrhythmia inducing active agent administered orally; sublingual opioid antagonist with the arrhythmia inducing active agent administered parenterally; sublingual opioid antagonist with the arrhythmia inducing active agent administered intranasally; sublingual opioid antagonist with the arrhythmia inducing active agent administered sublingually; and sublingual opioid antagonist with the arrhythmia inducing active agent administered transdermally.

Other routes of administration of the present invention include oral opioid antagonist with the arrhythmia inducing active agent administered orally; oral opioid antagonist with the arrhythmia inducing active agent administered parenterally; oral opioid antagonist with the arrhythmia inducing active agent administered intranasally; oral opioid antagonist with the arrhythmia inducing active agent administered sublingually; and oral opioid antagonist with the arrhythmia inducing active agent administered transdermally.

Other routes of administration of the present invention include parenteral opioid antagonist with the arrhythmia inducing active agent administered orally; parenteral opioid antagonist with the arrhythmia inducing active agent administered parenterally; parenteral opioid antagonist with the arrhythmia inducing active agent administered intranasally; parenteral opioid antagonist with the arrhythmia inducing active agent administered sublingually; and parenteral opioid antagonist with the arrhythmia inducing active agent administered transdermally.

In one embodiment, the opioid antagonist is administered in a transdermal system to provide, e.g., a dosing interval of 24 hours, a dosing interval of 3 days, or a dosing interval of 7 days.

In one embodiment, the opioid antagonist is administered sublingually. The opioid antagonist can be formulated in a sublingual formulation to provide, e.g., a dosing interval of 4 hours, a dosing interval of 6 hours, a dosing interval of 8 hours, a dosing interval of 12 hours, or a dosing interval of 24 hours.

In one embodiment, the opioid antagonist is administered in an oral dosage form to provide, e.g., a dosing interval of 4 hours, 6 hours, 8 hours, 12 hours or 24 hours.

In certain embodiments, the opioid agonist is oxycodone or a pharmaceutically acceptable salt thereof. In certain embodiments, the opioid agonist is oxycodone HC1.

In certain embodiments, the opioid agonist is hydrocodone or a pharmaceutically acceptable salt thereof. In certain embodiments, the opioid agonist is hydrocodone bitartrate.

In certain embodiments, the opioid agonist is hydromorphone or a pharmaceutically acceptable salt thereof. In certain embodiments, the opioid agonist is hydromorphone HC1.

In certain embodiments, the opioid agonist is oxymorphone or a pharmaceutically acceptable salt thereof. In certain embodiments, the opioid agonist is oxymorphone HC1.

In certain embodiments, the opioid agonist is morphine or a pharmaceutically acceptable salt thereof. In certain embodiments, the opioid agonist is morphine sulfate.

In certain embodiments, the opioid agonist is methadone or a pharmaceutically acceptable salt thereof. In certain embodiments, the opioid agonist is methadone HC1.

The opioid agonist may be formulated in the free base form, or as a pharmaceutically acceptable salt thereof.

The opioid agonist can be administered as a transdermal patch, a liquid oral dosage form, or as a solid oral dosage form in either immediate or controlled release form.

The opioid agonist can be administered in controlled release form with a dosing interval, e.g., of 8 hours, 12 hours or 24 hours. The opioid agonist can alternatively be administered in immediate release form with a dosing interval, e.g., of 2 hours, 4 hours, 6 hours or 8 hours. The opioid agonist, either in controlled release form or immediate release form, can be utilized in the present invention either alone or in combination with a non-opioid analgesic such as acetaminophen or an NSAID (e.g., aspirin, ibuprofen, naproxen, diclofenac, or a COX-2 inhibitor). Certain combination products can contain in addition to the opioid agonist, from 200 mg to 800 mg acetaminophen (e.g., 325 mg, 500 mg or 650 mg); from 200 mg to 800 mg aspirin (e.g., 325 mg or 500 mg); or 200 mg to 1000 mg ibuprofen (e.g., 200 mg, 400 mg, 600 mg or 800 mg).

The opioid agonist in controlled release form can be oxycodone hydrochloride in an amount, e.g., from 10 mg to 160 mg per unit dose. In specific embodiments, each unit dose can provide an amount of oxycodone hydrochloride of 10 mg, 20 mg, 30 mg, 40 mg, 50 mg, 60 mg, 70 mg, 80 mg, 100 mg, 120 mg or 160 mg. Controlled release oxycodone hydrochloride utilized in the present invention may be Oxycontin® (Oxycodone hydrochloride extended release tablets) commercially available from Purdue Pharma. The oxycodone hydrochloride in immediate release form can be in an amount from 2.5 mg to 50 mg, 2.5 mg, 4.5 mg; 4.8355 mg; 5 mg, 7.5 mg, 10 mg, 15 mg, 20 mg, or 30 mg. Immediate release oxycodone hydrochloride utilized in the present invention may be Tylox® (acetaminophen, oxycodone hydrochloride); Roxilox® (acetaminophen, oxycodone hydrochloride); Percocet® (acetaminophen, oxycodone hydrochloride); Oxycet® (acetaminophen, oxycodone hydrochloride); Roxicet® (acetaminophen, oxycodone hydrochloride); Percodan® (aspirin, oxycodone hydrochloride); Oxecta® (acetaminophen, oxycodone hydrochloride); or Roxicodone® (oxycodone hydrochloride).

The opioid agonist in controlled release form can be tramadol hydrochloride in an amount, e.g., from 100 mg to 300 mg per unit dose. In specific embodiments, each unit dose can provide an amount of tramadol hydrochloride of 100 mg, 150 mg, 200 mg, 250 mg, or 300 mg. Tramadol hydrochloride utilized in the present invention may be Conzip® (Tramadol hydrochloride extended release capsules); Ryzolt® (Tramadol hydrochloride extended release tablets); or Ultram ER® (Tramadol hydrochloride extended release capsules). Immediate release tramadol hydrochloride utilized in the present invention may be Ultracet® (acetaminophen, tramadol hydrochloride); or Rybix ODT® (tramadol hydrochloride orally disintegrating tablet).

The opioid agonist in controlled release form can be oxymorphone hydrochloride in an amount, e.g., from 5 mg to 40 mg per unit dose. In specific embodiments, each unit dose can provide an amount of oxymorphone hydrochloride of 5 mg, 10 mg, 15 mg, 20 mg, 25 mg, 30 mg, 35 mg or 40 mg. Oxymorphone hydrochloride utilized in the present invention may be Opana ER® (Oxymorphone hydrochloride extended release tablets). Immediate release oxymorphone hydrochloride utilized in the present invention may be Opana® (oxymorphone hydrochloride).

The opioid agonist in controlled release form can be hydrocodone bitartrate in an amount, e.g., from 2 mg to 200 mg per unit dose. In specific embodiments, each unit dose can provide an amount of hydrocodone bitartrate of 20 mg, 30 mg, 40 mg, 60 mg, 80 mg, 100 mg or 120 mg. Immediate release hydrocodone bitartrate utilized in the present invention may be Vicodin® (acetaminophen, hydrocodone bitartrate); Zydone® (acetaminophen, hydrocodone bitartrate); Anexsia® (acetaminophen, hydrocodone bitartrate); Lortab® (acetaminophen, hydrocodone bitartrate) or Vicoprofen® (ibuprofen, hydrocodone bitartrate).

The opioid agonist in controlled release form can be morphine sulfate in an amount, e.g., from 2 mg to 200 mg per unit dose. In specific embodiments, each unit dose can provide an amount of morphine sulfate of 15 mg, 20 mg, 30 mg, 40 mg, 60 mg, 80 mg, 100 mg, 120 mg or 200 mg. Morphine sulfate utilized in the present invention may be Avinza® (Morphine sulfate extended release capsules); Kadian® (Morphine sulfate extended release capsules); or MS Contin® (Morphine sulfate extended release tablets).

The opioid agonist in controlled release form can be hydromorphone hydrochloride in an amount, e.g., from 2 mg to 200 mg per unit dose. In specific embodiments, each unit dose can provide an amount of hydromorphone hydrochloride of 8 mg, 12 mg, 16 mg, 32 mg, 64 mg, or 128 mg; or 20 mg, 30 mg, 40 mg, 60 mg, 80 mg, 100 mg or 120 mg. Hydromorphone hydrochloride utilized in the present invention may be Exalgo® (Hydromorphone hydrochloride extended-release tablets); Palladone® (Hydromorphone hydrochloride extended-release capsules); or Dilaudid® (Hydromorphone hydrochloride oral tablets).

The opioid agonist in controlled release form can be tapentadol hydrochloride in an amount, e.g., from 2 mg to 400 mg per unit dose. In specific embodiments, each unit dose can provide an amount of tapentadol hydrochloride of 50 mg, 100 mg, 150 mg, or 250 mg. Tapentadol utilized in the present invention may be Nucynta ER® (Tapentadol extended release oral tablets) or Nucynta® (Tapentadol oral tablets).

The opioid agonist can be methadone hydrochloride in an amount, e.g., from 2.5 mg to 100 mg per unit dose. In specific embodiments, each unit does can provide an amount of methadone hydrochloride of 2.5 mg, 5 mg, 10 mg, 20 mg, 40 mg or 50 mg. The dosage form may be an oral solid dosage form (e.g., a tablet or capsule), a solution, a suspension or a parenteral. Methadone in the present invention may be Dolophine® (methadone hydrochloride tablets); Methadose® (methadone hydrochloride tablet); or Diskets® (methadone hydrochloride tablet). The methadone can be a liquid concentrate for oral use (e.g., 10 mg/mL), an injectable solution (e.g., lOmg/mL), an oral solution (e.g., 10mg/5mL or 5mg/5mL), a tablet for oral suspension (e.g., 40 mg) or an oral tablet (e.g., 5 mg or 10 mg).

The opioid agonist can be fentanyl disposed in a transdermal system that delivers the fentanyl in an amount, e.g., of 12.5 mcg/hr; 25 mcg/hr; 50 mcg/hr; 75 mcg/hr or 100 mcg/hr. Fentanyl utilized in the present invention can be Duragesic® (fentanyl film, extended release).

In certain embodiments, the opioid antagonist is administered orally concurrently with oral administration of the arrhythmia inducing active agent. The opioid antagonist can be in the same oral dosage form as the arrhythmia inducing active agent or can be in a separate oral dosage form as the arrhythmia inducing active agent.

The opioid antagonist and the arrhythmia inducing active agent can both be formulated to provide (i) an immediate release from the same or different oral dosage forms or (ii) controlled release from the same or different dosage forms.

In alternate embodiments, the opioid antagonist can be formulated for immediate release and the arrhythmia inducing active agent can be formulated for controlled release, from the same or different oral dosage forms.

In further embodiments, the opioid antagonist can be formulated for controlled release and the arrhythmia inducing active agent can be formulated for immediate release, from the same or different oral dosage forms.

Preferably, the oral dosage form containing the opioid antagonist, the arrhythmia inducing active agent, or both agents, is in the form of a tablet or capsule.

In formulations containing both agents, the opioid antagonist and the arrhythmia inducing active agent can be commingled in a tablet or capsule.

In a tablet formulation, the core can contain the opioid antagonist which is layered with a coating of the arrhythmia inducing active agent. Alternatively, the core can contain the arrhythmia inducing active agent which is layered with a coating of the opioid antagonist. In other embodiments, the formulation can be in a laminar arrangement such that the opioid antagonist and the arrhythmia inducing active agent are layered in at least a bilayer tablet.

In capsule formulations, the agents can be in the same multiparticulate formulation or in separate multiparticulate formulations that are contained in a pharmaceutically acceptable capsule (e.g., a gelatin capsule). The components of the multiparticulate formulation can be in the form of a core containing the opioid antagonist which is layered with a coating of the arrhythmia inducing active agent. Alternatively, the components of the multiparticulate formulation can be in the form of a core containing the arrhythmia inducing active agent which is layered with a coating of the opioid antagonist. In other embodiments, the capsule can contain a granulation or powder blend containing both the opioid antagonist and the arrhythmia inducing active agent, or separate granulations or powders each containing the opioid antagonist or the arrhythmia inducing active agent.

In oral formulations, the opioid antagonist and/or the arrhythmia inducing active agent can be formulated to provide a delayed release in order to target release at a specific site in the gastro-intestinal tract (e.g., the intestine or the colon). The delayed release can be obtained with an enteric coating on the tablet, multiparticulates, capsule or any other dosage form or component of a dosage form, as appropriate. Enteric materials that can be utilized to provide a delayed release of opioid antagonist and/or the arrhythmia inducing active agent include, e.g., shellac, cellulose acetate phthalate (CAP), polyvinyl acetate phthalate (PVAP), hydroxypropylmethylcellulose phthalate, methacrylic acid ester copolymers and zein.

The opioid antagonist can be in an amount that (i) does not cause a decrease in the analgesic effectiveness of the opioid agonist, or (ii) does not cause a substantial decrease in the analgesic effectiveness of the opioid agonist, or (iii) provides an increase in analgesia as compared to the administration of the opioid agonist alone.

The concentration of opioid antagonist that affects the analgesic efficacy of the concurrently administered opioid agonist as compared to the concentration of opioid antagonist that prevents or treats opioid induced arrhythmia depends on the identity of the opioid agonist that is concurrently being administered. Preferably, the window of separation is sufficient such that the opioid antagonist effectively prevents or treats the opioid induced arrhythmia without affecting the analgesic potency of the opioid.

### FORMULATIONS OF OPIOID ANTAGONIST AND THE ARRHYTHMIA INDUCING ACTIVE AGENT

The opioid antagonist and/or the arrhythmia inducing active agent can be administered as a component of a pharmaceutical composition that comprises a pharmaceutically acceptable carrier or excipient. The opioid antagonist and/or the arrhythmia inducing active agent can be formulated as (i) separate formulations intended for different routes of administration, (ii) separate formulations intended for the same route of administration, or (iii) in the same formulation to be administered together by the same route of administration. The pharmaceutical compositions can be administered by any appropriate route, as determined by the medical practitioner. Methods of administration may include intradermal, intramuscular, intraperitoneal, parenteral, intravenous, subcutaneous, intranasal, epidural, oral, sublingual, buccal, intracerebral, intravaginal, transdermal, transmucosal, rectal, by inhalation, or topical (particularly the skin).

Pharmaceutical compositions of the invention can take the form of solutions, suspensions, emulsions, tablets, pills, pellets, multi-particulates, capsules, capsules containing liquids, capsules containing powders, capsules containing multi-particulates, lozenges, sustained-release formulations, suppositories, aerosols, sprays, or any other form suitable for use. In one embodiment, the composition is in the form of a capsule (see, e.g., U.S. Pat. No. 5,698,155). Other examples of suitable pharmaceutical excipients are described in Remington's Pharmaceutical Sciences 1447-1676 (Alfonso R. Gennaro ed., 19th ed. 1995).

Pharmaceutical compositions of the invention preferably comprise a suitable amount of a pharmaceutically acceptable excipient so as to provide the form for proper administration to the patient. Such a pharmaceutical excipient can be a diluent, suspending agent, solubilizer, binder, disintegrant, buffer, glidant, preservative, coloring agent, lubricant. The pharmaceutical excipient can be a liquid, such as water or an oil, including those of petroleum, animal, vegetable, or synthetic origin, such as peanut oil, soybean oil, mineral oil, sesame oil. The pharmaceutical excipient can be saline, gum acacia, gelatin, starch paste, talc, keratin, colloidal silica, urea. In addition, auxiliary, stabilizing, thickening, lubricating, and coloring agents can be used. In one embodiment, the pharmaceutically acceptable excipient is sterile when administered to a patient. Water is a particularly useful excipient when the pharmaceutical composition is administered intravenously. Saline solutions and aqueous dextrose and glycerol solutions can also be employed as liquid excipients, particularly for injectable solutions. Suitable pharmaceutical excipients also include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol. The invention compositions, if desired, can also contain minor amounts of wetting or emulsifying agents, or pH buffering agents. Specific examples of pharmaceutically acceptable carriers and excipients that can be used to formulate oral dosage forms are described in the Handbook of Pharmaceutical Excipients, American Pharmaceutical Association (1986).

In certain embodiments, the pharmaceutical compositions are formulated for oral administration. A pharmaceutical composition of the invention to be orally delivered can be in the form of tablets, capsules, gelcaps, caplets, lozenges, aqueous or oily solutions, suspensions, granules, powders, emulsions, syrups, or elixirs, for example. When the opioid antagonist and/or the arrhythmia inducing active agent are incorporated into oral tablets, such tablets can be compressed, tablet triturates, enteric -coated, sugar-coated, film-coated, multiply compressed or multiply layered.

An orally administered pharmaceutical composition can contain one or more additional agents such as, for example, sweetening agents such as fructose, aspartame or saccharin; flavoring agents such as peppermint, oil of wintergreen, or cherry; coloring agents; and preserving agents, and stabilizers, to provide stable, pharmaceutically palatable dosage forms. Techniques and compositions for making solid oral dosage forms are described in Pharmaceutical Dosage Forms: Tablets (Lieberman, Lachman and Schwartz, eds., 2nd ed.) published by Marcel Dekker, Inc. Techniques and compositions for making tablets (compressed and molded), capsules (hard and soft gelatin) and pills are also described in Remington's Pharmaceutical Sciences 1553-1593 (Arthur Osol, ed., 16.sup.th ed., Mack Publishing, Easton, Pa. 1980). Liquid oral dosage forms include aqueous and nonaqueous solutions, emulsions, suspensions, and solutions and/or suspensions reconstituted from non-effervescent granules, optionally containing one or more suitable solvents, preservatives, emulsifying agents, suspending agents, diluents, sweeteners, coloring agents, flavoring agents. Techniques and compositions for making liquid oral dosage forms are described in Pharmaceutical Dosage Forms: Disperse Systems, (Lieberman, Rieger and Banker, eds.) published by Marcel Dekker, Inc.

When the opioid antagonist and/or the arrhythmia inducing active agent is formulated for parenteral administration by injection (e.g., continuous infusion or bolus injection), the formulation can be in the form of a suspension, solution, or emulsion in an oily or aqueous vehicle, and such formulations can further comprise pharmaceutically necessary additives such as one or more stabilizing agents, suspending agents, dispersing agents. When the opioid antagonist and/or the arrhythmia inducing active agent is to be injected parenterally, it can be, e.g., in the form of an isotonic sterile solution. The opioid antagonist and/or the arrhythmia inducing active agent can also be in the form of a powder for reconstitution as an injectable formulation.

In certain embodiments, the opioid antagonist and/or the arrhythmia inducing active agent is formulated into a pharmaceutical composition for intravenous administration. Typically, such compositions comprise sterile isotonic aqueous buffer. Where necessary, the compositions can also include a solubilizing agent. A pharmaceutical composition for intravenous administration can optionally include a local anesthetic such as benzocaine or prilocaine to lessen pain at the site of the injection. Generally, the ingredients are supplied either separately or mixed together in unit dosage form, for example, as a dry lyophilized powder or water-free concentrate in a hermetically sealed container such as an ampule or sachet indicating the quantity of active agent. Where the opioid antagonist and/or the arrhythmia inducing active agent is to be administered by infusion, it can be dispensed, for example, with an infusion bottle containing sterile pharmaceutical grade water or saline. When the opioid antagonist and/or the arrhythmia inducing active agent is administered by injection, an ampule of sterile water for injection or saline can be provided so that the ingredients can be mixed prior to administration.

When the opioid antagonist and/or the arrhythmia inducing active agent is to be administered by inhalation, it can be formulated into a dry aerosol, or an aqueous or partially aqueous solution.

In another embodiment, the opioid antagonist and/or the arrhythmia inducing active agent can be delivered in a vesicle, in particular a liposome (see Langer, Science 249: 1527- 1533 (1990); and Treat et al., Liposomes in the Therapy of Infectious Disease and Cancer 317-327 and 353-365 (1989)).

In certain embodiments, the opioid antagonist and/or the arrhythmia inducing active agent can be delivered in an immediate release form. In other embodiments, the opioid antagonist and/or the arrhythmia inducing active agent can be delivered in a controlled- release system or sustained-release system. Controlled- or sustained-release pharmaceutical compositions can have a common goal of improving drug therapy over the results achieved by their non-controlled or non-sustained-release counterparts. Advantages of controlled- or sustained-release compositions include extended activity of the drug, reduced dosage frequency, and increased compliance. In addition, controlled- or sustained-release compositions can favorably affect the time of onset of action or other characteristics, such as blood levels of the opioid antagonist and/or the arrhythmia inducing active agent, and can thus reduce the occurrence of adverse side effects.

Controlled- or sustained-release compositions can initially release an amount of the opioid antagonist and/or the arrhythmia inducing active agent that promptly produces the desired therapeutic or prophylactic effect, and gradually and continually release other amounts of the opioid antagonist and/or the arrhythmia inducing active agent to maintain a level of therapeutic or prophylactic effect over an extended period of time. To maintain a constant level of the opioid antagonist and/or the arrhythmia inducing active agent in the body, the pharmaceutical composition can release the active(s) from the dosage form at a rate that will replace the amount of active(s) being metabolized and excreted from the body. Controlled or sustained release of an active ingredient can be triggered by any of various conditions, including changes in pH, changes in temperature, concentration or availability of enzymes, concentration or availability of water, or other physiological conditions or compounds.

Controlled-release and sustained-release means for use according to the present invention may be selected from those known in the art. Examples include those described in U.S. Pat. Nos. 3,845,770; 3,916,899; 3,536,809; 3,598,123; 4,008,719; 5,674,533; 5,059,595; 5,591,767; 5,120,548; 5,073,543; 5,639,476; 5,354,556; and 5,733,566. Such dosage forms can be used to provide controlled- or sustained-release of one or both of the active ingredients using, for example, hydropropylmethyl cellulose, other polymer matrices, gels, permeable membranes, osmotic systems, multilayer coatings, microparticles, multiparticulates, liposomes, microspheres, or a combination thereof to provide the desired release profile in varying proportions. Suitable controlled- or sustained-release formulations known in the art, including those described herein, can be readily selected for use with the active ingredients of the invention in view of this disclosure. See also Goodson, "Dental Applications" (pp. 115-138) in Medical Applications of Controlled Release, Vol. 2, Applications and Evaluation, R. S. Langer and D. L. Wise eds., CRC Press (1984). Other controlled- or sustained-release systems that are discussed in the review by Langer, Science 249: 1527-1533 (1990) can be selected for use according to the present invention. In one embodiment, a pump can be used (Langer, Science 249: 1527-1533 (1990); Sefton, CRC Crit. Ref Biomed. Eng. 14:201 (1987); Buchwald et al., Surgery 88:507 (1980); and Saudek et al., N. Engl. J. Med. 321:574 (1989)). In another embodiment, polymeric materials can be used (see Medical Applications of Controlled Release (Langer and Wise eds., 1974); Controlled Drug Bioavailability, Drug Product Design and Performance (Smolen and Ball eds., 1984); Ranger and Peppas, J. Macromol. Sci. Rev. Macromol. Chem. 23:61 (1983); Levy et al., Science 228: 190 (1985); During et al., Ann. Neurol. 25:351 (1989); and Howard et al., J. Neurosurg. 71 : 105 (1989)).

When in tablet or pill form, a pharmaceutical composition of the invention can be coated to delay disintegration and absorption in the gastrointestinal tract thereby providing targeted release to a particular portion of the GI tract, or providing a sustained action over an extended period of time. Selectively permeable membranes surrounding an osmotically active driving compound are also suitable for orally administered compositions. In these latter platforms, fluid from the environment surrounding the capsule is imbibed by the driving compound, which swells to displace the agent or agent composition through an aperture. These delivery platforms can provide an essentially zero order delivery profile as opposed to the spiked profiles of immediate release formulations. A time-delay material such as glycerol monostearate or glycerol stearate can also be used. Oral compositions preferably include standard excipients of pharmaceutical grade selected, for example, from mannitol, lactose, starch, magnesium stearate, sodium saccharin, cellulose, and magnesium carbonate, among others.

Controlled release oral dosage forms according to the present invention may also be prepared as osmotic dosage forms. The osmotic dosage forms preferably include a bilayer core comprising a drug layer (containing the opioid antagonist and/or the arrhythmia inducing active agent) and a delivery or push layer, wherein the bilayer core is surrounded by a semipermeable wall and optionally having at least one passageway disposed therein.

The expression "passageway" as used for the purpose of this invention, includes an aperture, orifice, bore, pore, porous element, fiber, capillary tube, porous overlay, porous insert, microporous member, or porous composition through any of which the opioid antagonist and/or the opioid agonist can diffuse, migrate or be pumped through. The passageway can also include a compound that erodes or is leached from the wall in the fluid environment of use to produce at least one passageway. Representative compounds for forming a passageway include erodible poly(glycolic) acid or poly(lactic) acid in the wall; a gelatinous filament; a water-removable poly(vinyl alcohol); and leachable compounds such as fluid-removable pore-forming polysaccharides, acids, salts or oxides. Examples of leachable compounds include sorbitol, sucrose, lactose, maltose, or fructose. The passageway can have any shape, such as round, triangular, square and elliptical, for assisting in the controlled release of the opioid antagonist and/or the opioid agonist from the dosage form. The dosage form can be manufactured with one or more passageways in spaced-apart relation on one or more surfaces of the dosage form. A passageway and equipment for forming a passageway are described in U.S. Pat. Nos. 3,845,770; 3,916,899; 4,063,064 and 4,088,864. Passageways prepared by leaching are described in U.S. Pat. Nos. 4,200,098 and 4,285,987.

In certain embodiments the drug layer may comprise at least one polymer hydrogel. Examples of polymer hydrogels include a maltodextrin polymer; a poly(alkylene oxide) such as a poly(ethylene oxide) and a poly(propylene oxide); an alkali carboxyalkylcellulose, wherein the alkali is sodium or potassium and the alkyl is methyl, ethyl, propyl, or butyl; and a copolymer of ethylene- acrylic acid, including methacrylic and ethacrylic acid.

In certain embodiments of the present invention, the delivery or push layer comprises an osmopolymer. Examples of an osmopolymer include a member selected from the group consisting of a polyalkylene oxide and a carboxyalkylcellulose. The polyalkylene oxide may be a member selected from the group consisting of polymethylene oxide, polyethylene oxide and polypropylene oxide. The carboxyalkylcellulose may be a member selected from the group consisting of alkali carboxyalkylcellulose, sodium carboxymethylcellulose, potassium carboxymethylcellulose, sodium carboxyethylcellulose, lithium carboxymethylcellulose, sodium carboxyethylcellulose, carboxyalkylhydroxyalkylcellulose, carboxymethylhydroxyethylcellulose, carboxyethylhydroxyethylcellulose and carboxymethylhydroxypropylcellulose. The osmopolymers used for the displacement layer exhibit an osmotic pressure gradient across the semipermeable wall. The osmopolymers imbibe fluid into the dosage form, thereby swelling and expanding as an osmotic hydrogel, whereby they push the contents of the drug layer from the osmotic dosage form.

The push layer may also include one or more osmotically effective compounds that imbibe an environmental fluid, for example, from the gastrointestinal tract, into the dosage form to contribute to the delivery kinetics of the displacement layer. Examples of osmotically effective compounds comprise a member selected from the group consisting of osmotic salts and osmotic carbohydrates. Examples of specific osmagents include sodium chloride, potassium chloride, magnesium sulfate, lithium phosphate, lithium chloride, sodium phosphate, potassium sulfate, sodium sulfate, potassium phosphate, glucose, fructose and maltose.

The push layer may optionally include a hydroxypropylalkylcellulose such as hydroxypropylmethylcellulose, hydroxypropylethylcellulose, hydroxypropyl isopropyl cellulose, hydroxypropylbutylcellulose, and hydroxypropylpentylcellulose.

In certain alternative embodiments, the dosage form comprises a substantially homogenous core comprising the opioid antagonist and/or the opioid agonist, a pharmaceutically acceptable polymer (e.g., polyethylene oxide) and optional excipients such as disintegrants and absorption enhancers. The substantially homogenous core is surrounded by a semipermeable wall having a passageway (as defined above) for the release of the opioid antagonist and/or the opioid agonist. Such an embodiment would not require a push layer.

In certain embodiments, the semipermeable wall comprises a member selected from the group consisting of a cellulose ester polymer, a cellulose ether polymer and a cellulose ester-ether polymer. Representative wall polymers comprise a member selected from the group consisting of cellulose acylate, cellulose diacylate, cellulose triacylate, cellulose acetate, cellulose diacetate, cellulose triacetate, mono-, di- and tricellulose alkenylates, and mono-, di- and tricellulose alkynylates.

With osmotic systems, the opioid antagonist or the arrhythmia inducing active agent can be formulated for controlled release and the other agent can be formulated for immediate release, e.g., by coating onto the semipermeable wall.

Pharmaceutical compositions of the invention include single unit dosage forms suitable for oral administration such as tablets, capsules, gelcaps, and caplets, which may be adapted for controlled or immediate release.

In certain embodiments, both the opioid antagonist and the arrhythmia inducing active agent can be included in the same dosage form. For example, the opioid antagonist and the arrhythmia inducing active agent can both be included in a transdermal dosage form such that each agent is administered according to the desired rate. In certain embodiments, the two agents can be segregated from each other in a dual reservoir system.

### TRANSDERMAL DOSAGE FORMS

In certain embodiments, wherein the opioid antagonist and/or the arrhythmia inducing active agent are administered in a transdermal device, the formulation can, e.g., be a transdermal patch, a transdermal plaster, a transdermal disc or an iontophoretic transdermal device.

Transdermal dosage forms used in accordance with the invention can include a backing layer made of a pharmaceutically acceptable material which is impermeable to the opioid antagonist and/or the arrhythmia inducing active agent. The backing layer can serve as a protective cover for the opioid antagonist and/or the arrhythmia inducing active agent and may also provide a support function. Examples of materials suitable for making the backing layer are films of high and low density polyethylene, polypropylene, polyvinylchloride, polyurethane, polyesters such as poly(ethylene phthalate), metal foils, metal foil laminates of suitable polymer films, textile fabrics. The backing layer can be any appropriate thickness which will provide the desired protective and support functions. A suitable thickness can be, e.g., from 10 microns to 200 microns.

In certain embodiments, the transdermal dosage forms used in accordance with the invention contain a biologically acceptable polymer matrix layer. Generally, the polymers used to form the polymer matrix layer are capable of allowing the opioid antagonist and/or the arrhythmia inducing active agent to pass through at a controlled rate. A list of exemplary materials for inclusion in the polymer matrix includes polyethylene, polypropylene, ethylene/propylene copolymers, ethylene/ethylacrylate copolymers, ethylenevinyl acetate copolymers, silicones, natural or synthetic rubber, polyacrylic esters and copolymers thereof, polyurethanes, polyisobutylene, chlorinated polyethylene, polyvinylchloride, vinyl chloride- vinyl acetate copolymer, polymethacrylates, polyvinylidene chloride, poly (ethylene terephthalate), ethylene- vinyl alcohol copolymer, ethylene-vinyloxyethanol copolymer, silicones, silicone copolymers such as polysiloxane-polymethacrylate copolymers, cellulose polymers (e.g., ethyl cellulose, and cellulose esters), polycarbonates, polytetrafluoroethylene and mixtures thereof.

The polymer matrix layer may optionally include a pharmaceutically acceptable cross-linking agent such as, e.g., tetrapropoxy silane.

In certain embodiments, the transdermal delivery systems used in accordance with the methods of the present invention include an adhesive layer to affix the dosage form to the skin of the patient for a desired period of administration, e.g., 1 day, 2 days, 3 days, 4 days, 5 days, 6 days, or 7 days. If the adhesive layer of the dosage form fails to provide adhesion for the desired period of time, it is possible to maintain contact between the dosage form with the skin, e.g., by affixing the dosage form to the skin of the patient with an adhesive tape.

The adhesive layer may include an adhesive such as polyacrylic adhesive polymers, acrylate copolymers (e.g., polyacrylate) and polyisobutylene adhesive polymers.

The transdermal dosage forms which can be used in accordance with the present invention may optionally include a permeation enhancing agent. Permeation enhancing agents are compounds which promote penetration and/or absorption of the opioid antagonist and/or the arrhythmia inducing active agent into the blood stream of the patient. Permeation enhancing agents include polyethylene glycols and surfactants.

In one embodiment, the transdermal dosage form which may be used in accordance with the present invention includes a non-permeable backing layer comprising, e.g., a polyester; an adhesive layer comprising, e.g., a polyacrylate; and a matrix containing the opioid antagonist and other excipients such as softeners, permeability enhancers, viscosity agents.

The opioid antagonist and/or the arrhythmia inducing active agent may be included in the device in a drug reservoir, drug matrix or drug/adhesive layer.

Certain preferred transdermal delivery systems also include a softening agent. Suitable softening agents include higher alcohols such as dodecanol, undecanol, octanol, esters of carboxylic acids, diesters of dicarboxylic acids and triglycerides. Further examples of suitable softeners are multivalent alcohols such as levulinic acid, caprylic acids, glycerol and 1, 2-propanediol, which can also be etherified by a polyethylene glycol.

A drug solvent may also be included in the transdermal delivery systems of the present invention. A non-limiting list of suitable solvents includes those with at least one acidic group such as monoesters of dicarboxylic acids (e.g., monomethylglutarate and monomethyladipate) .

In certain embodiments, the transdermal dosage form includes a removable protective layer. The removable protective layer is removed prior to application, and may comprise the materials used for the production of the backing layer disclosed above provided that they are rendered removable, e.g., by silicone treatment. Other removable protective layers include polytetra-fluoroethylene, treated paper, allophane, polyvinyl chloride. Generally, the removable protective layer is in contact with the adhesive layer and provides a convenient means of maintaining the integrity of the adhesive layer until the desired time of application.

The transdermal system utilized in the present invention is used by adhering the transdermal system to a dermal surface of a patient. The dermal surface should be clean and unbroken. In certain embodiments, the transdermal system will be sufficiently adhesive to remain adhered to the patient's skin during normal everyday activities and for an adequate period of time. In other embodiments, it may be necessary to further secure the transdermal system to the patient, e.g., by wrapping tape or a medical bandage around the area to which the transdermal system has been applied.

In some embodiments, the transdermal system can be cut or otherwise separated into two or more separate pieces to adjust the amount of opioid antagonist and or arrhythmia inducing active agent that will be delivered to the patient. For example, the transdermal system may include perforations or lines along which to cut for dividing the transdermal system into multiple doses.

### MUCOSAL TABLETS AND FILMS

In certain embodiments, the opioid antagonist and/or the arrhythmia inducing active agent can be formulated for application to the mucosal tissue. Such a formulation can be a tablet, film or spray adapted for lingual (i.e., to be placed onto the tongue), sublingual (i.e., to be placed under the tongue), buccal (i.e., to be applied to the cheek), or gingival (i.e., to be applied to the gums) administration. One benefit of such administration is the avoidance or reduction of first pass metabolism associated with oral administration.

Sublingual, lingual, buccal and gingival tablets, and films are formulated to disintegrate rapidly in the mouth to provide absorption of the opioid antagonist in the oral cavity in a relatively short period of time. Such forms may contain soluble excipients such as lactose, mannitol, dextrose, sucrose or mixtures thereof. Such forms may also contain granulating and disintegrating agents such as starch, silicon dioxide, or sodium starch glycolate, binding agents such as povidone or hydroxypropyl-methyl cellulose and lubricating agents such as magnesium stearate. Such forms may also comprise a bioerodible polymeric carrier that optionally may also serve to adhere the dosage form to the sublingual, lingual, buccal, or gingival mucosa.

In some embodiments, the opioid antagonist and/or the arrhythmia inducing active agent can be formulated as a gel in the form of a film or strip. The film should be capable of disintegrating quickly, e.g., in 0.5 second to 120 seconds from contact with a surface in the oral cavity. In certain embodiments, the film is capable of disintegrating within 0.5 second to 60 seconds, or in less than 5 seconds, or in less than 10 seconds, or in less than 15 seconds, or in less than 20 seconds, or in less than 30 seconds, or in less than 45 seconds.

The film may comprise hydrophilic (water-soluble and water- swellable) polymers that adhere to a wet surface in the oral cavity. Polymeric carriers may be selected from acrylic acid polymers, hydrolyzed polyvinylalcohols, polyethylene oxides, polyacrylates, vinyl polymers, polyvinylpyrrolidones, dextrans, guar gums, pectins; starches, and cellulosic polymers, among others.

Mucosal tablets or films can also include a permeation enhancer to increase the rate at which the opioid antagonist and/or the arrhythmia inducing active agent permeate through the mucosal tissue to which it is applied, e.g., the buccal, lingual, gingival, or sublingual mucosa. Permeation enhancers may be selected from dimethylsulfoxide ("DMSO"), dimethyl formamide ("DMF"), N,N-dimethylacetamide ("DMA"), decylmethylsulfoxide ("CioMSO"), polyethylene glycol monolaurate ("PEGML"), glycerol monolaurate, lecithin, 1-substituted azacycloheptan-2-ones, alcohols, and surfactants, among others.

The following examples are set forth to assist in understanding the invention.

### Examples

### Example 1

A QT study following E14 principles regarding design, conduct, analysis, and interpretation was conducted with the following objectives

### Primary:

To evaluate the ECG effects of 10, 40, and 80 mcg/hr buprenorphine delivered by a Buprenorphine Transdermal System (BTDS) alone, or by BTDS dosed with naltrexone tablets, relative to placebo in healthy male and female subjects

### Secondary:

To evaluate the ECG effects of moxifloxacin relative to placebo (assay sensitivity) To evaluate the ECG effects of BTDS + Naltrexone (NTX) vs. BTDS alone
To evaluate the ECG effects of naltrexone alone vs. placebo

The study was a randomized, double-blind, placebo and positive controlled, parallel group study with the following parameters:

### Key Inclusion/Exclusion Criteria

Healthy male or female subjects aged 18 to 55 years, inclusive
Heart rate between 45-85 bpm
No clinically significant medical history or disease
Subjects with personal or family history of prolonged QT interval or abnormal cardiovascular condition excluded

### Sample size

60 subjects/treatment group
Single study center, multiple study cohorts studied successively

### Treatments

1) BTDS alone (Butrans® 5, 10, 20, 40, 80 mcg/hr)
2) Moxifloxacin (400 mg single doses on Days 6, 13, 17)
3) Placebo
4) Naltrexone alone (NTX 50 mg ql2h days 4-17)
5) BTDS + Naltrexone (BTDS 10, 20, 40, 80 mcg/hr with NTX 50 mg ql2h days 4-17)

BTDS doses of 40 and 80 mcg/hr were achieved with multiple 20 mcg/hr patches and there were matching placebos for each active dose form
BTDS or matching placebo patches were applied according to a dose-escalation and dose tapering scheme
24-hr ECG were collected at steady-state for each BTDS dose level at Days 6 (10 mcg/hr), 13 (40 mcg/hr) and 17 (80 mcg/hr).

Pharmacokinetic samples were collected at the beginning, mid-point, and end of each on-treatment 24-hour ECG collection.

Continuous 24-hr 12-lead digital Holter ECG collections were performed as follows:
- Two 24-hour baseline recordings (Days -2 and -1)
- Three 24-hour periods on-treatment for Days 6 (10 mcg/hr), 13 (40 mcg/hr) and 17 (80 mcg/hr).

ECGs were extracted at 13 time points (0, 0.5, 1, 1.5, 2, 2.5, 3, 4, 7, 10, 13, 18, 23.5 hours) over each 24-hour ECG recording period.

Subjects rested supine for at least 10 minutes prior to each nominal ECG extraction time and activity was limited for the prior 2 hours.

4 ECGs were extracted and quantified at each nominal time point by a blinded central ECG laboratory. The primary QT correction for heart rate was QTcI. 104 baseline ECGs were used to determine subject-specific HR correction.

Placebo-corrected change from baseline QTcI means and 90% CIs were calculated using a mixed effects general linear model with covariates for time, treatment, time by treatment interaction, and sex.

The results depicted in Table 1 are preliminary findings from draft TLGs.

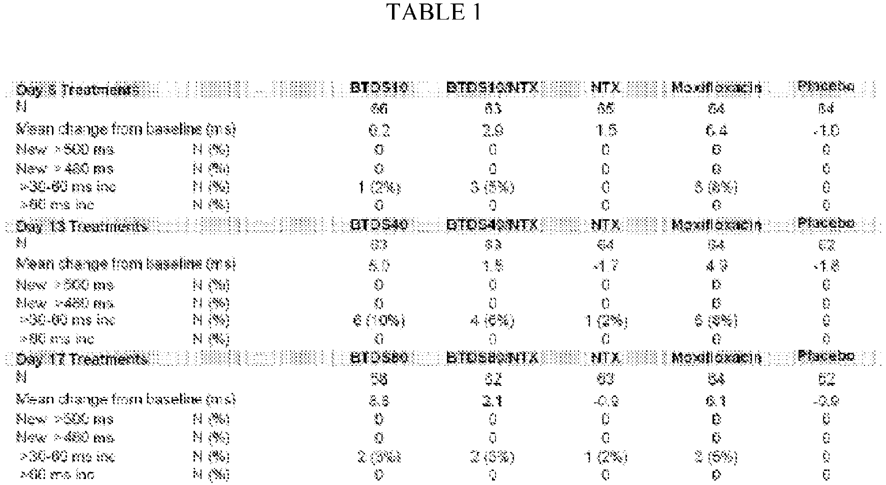

### STUDY VALIDITY AND SENSITIVITY

Review of the effects of treatments on heart rate, blood pressure, pulse rate, QRS intervals and ECG morphology showed no clinically-significant findings.

Placebo QTcI results were as expected on each of Days 6, 13, and 17.
- Negative control performance acceptable
- Magnitude and time-course of QTcI changes match historical precedents

Moxifloxacin QTcI results were as expected on each of Days 6, 13, and 17.
- Positive control performance acceptable
- Magnitude and time-course of QTcI changes match historical precedents
- Assay sensitivity demonstrated

Diagnostics indicate valid and sensitive study for characterization of effects of BTDS, NTX, and BTDS+NTX on QTcI

### PRELIMINARY BUPRENORPHINE CONCLUSIONS

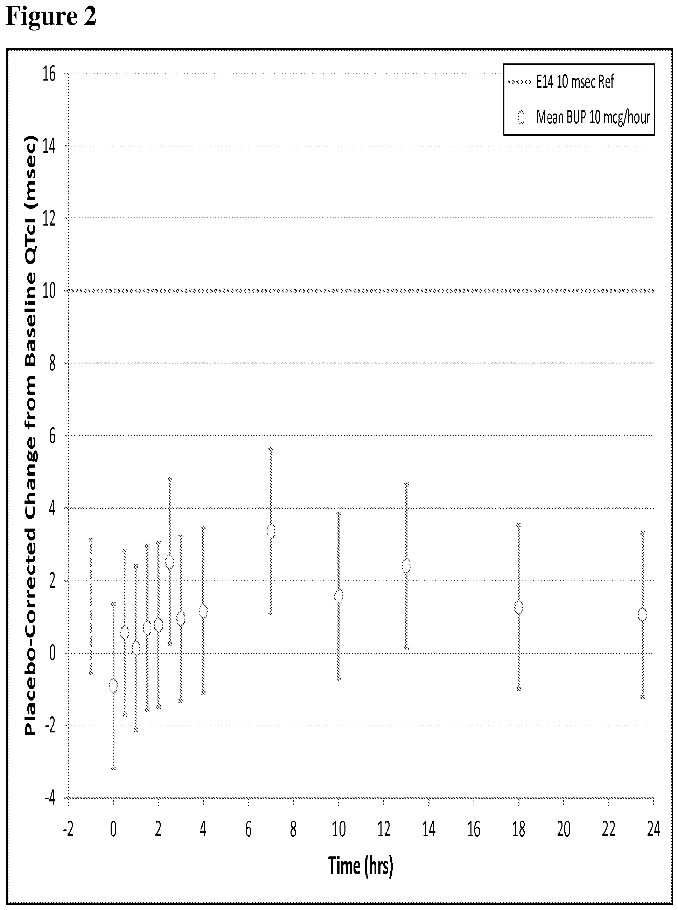

BTDS 10 results were negative (e.g., as shown in Figure 2).
- Time-matched CI upper bound < 6 ms at all 13 time points

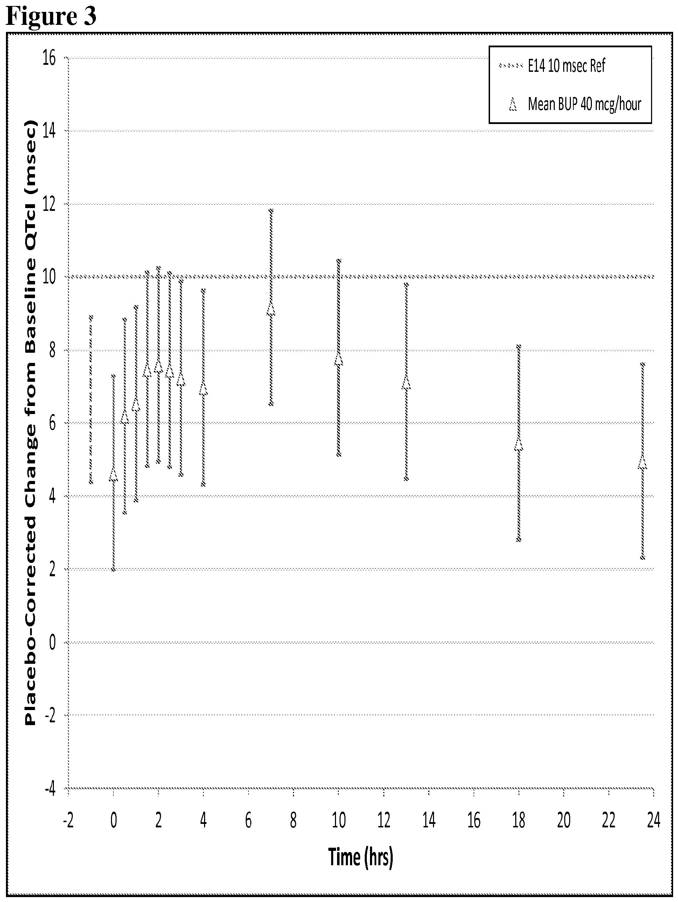

BTDS 40 showed small prolongation of QTcI (e.g., as shown in Figure 3).
- Time-averaged magnitude similar to Moxifloxacin
- Time-matched CI upper bound > 10 ms at 5 time points

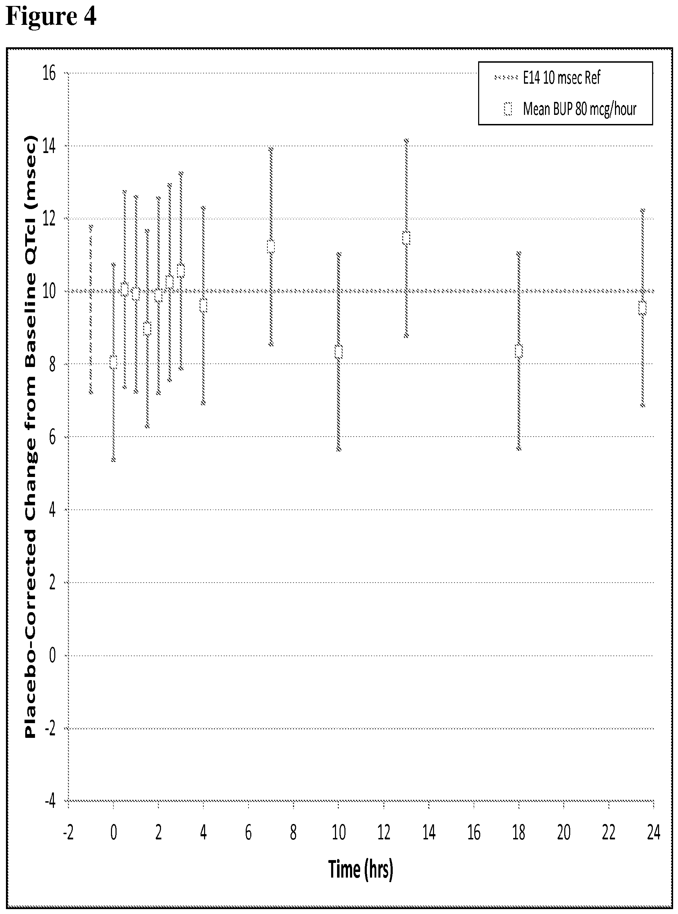

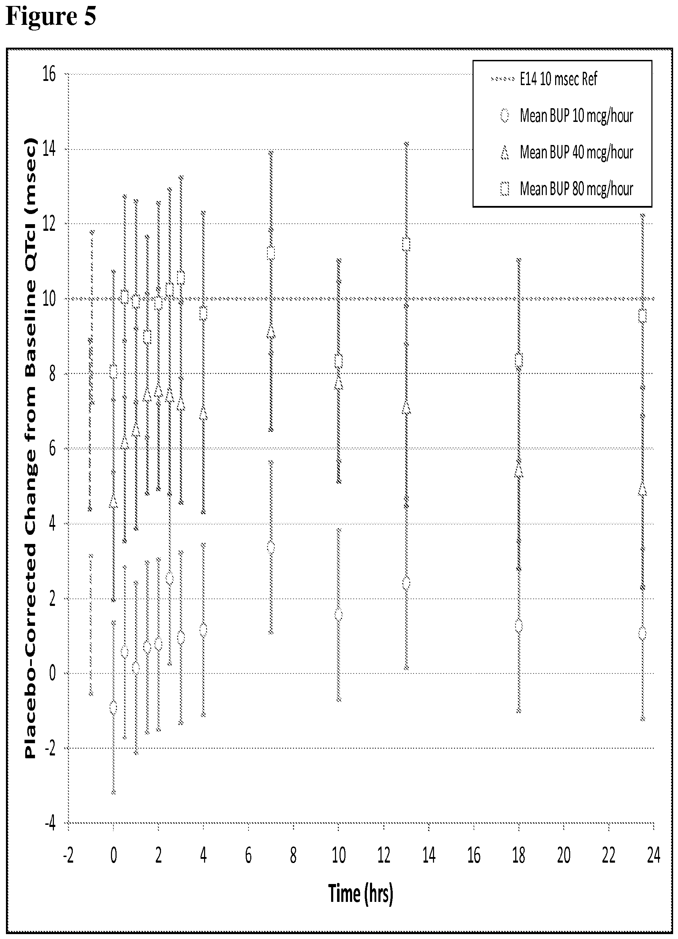

BTDS 80 showed dose-related increase in QTcI prolongation (e.g., as shown in Figure 4 and 5).
- Time averaged effect exceeds Moxifloxacin
- Mean effect > 10 ms at 5 time points
- CI upper bound > 10 ms at all 13 time points
- Time-matched maximum effect (Mean 11.4 ms, CI upper bound 14.1 ms)

As shown in Table 1, naltrexone administered with buprenorphine reduced the mean change in baseline of the QT interval as compared to buprenorphine alone. See, e.g., the data for Day 17 treatments where the change in baseline for BTDS 80 was 8.8 ms and the mean change in baseline for BTDS 80/NTX was 2.1 ms

The specific embodiments disclosed in the examples are intended as illustrations of a few aspects of the invention. Indeed, various modifications of the invention in addition to those shown and described herein will become apparent to those skilled in the art.

## Claims

1. Opioid antagonist for use in a method of preventing or treating an arrhythmia in a patient by lowering the QTcI in said patient to less than 10 ms, wherein the arrhythmia is induced by the administration of an opioid analgesic, the method comprising administering to a patient in need thereof an effective amount of the opioid antagonist concurrently with the opioid analgesic to prevent or treat the arrhythmia, wherein the opioid analgesic is selected from the group consisting of oxycodone, morphine, codeine, oxymorphone, fentanyl, hydrocodone, hydromorphone, tramadol, buprenorphine, methadone, and pharmaceutically acceptable salts thereof, and wherein the opioid antagonist is selected from the group consisting of naltrexone, naloxone, nalmefene, cyclazacine, levallorphan, and pharmaceutically acceptable salts thereof.

2. Opioid antagonist for use according to claim 1, wherein the arrhythmia is a prolonged QT interval.

3. Opioid antagonist for use according to claim 2, wherein the patient is diagnosed with ventricular tachyarrhythmia or with torsades de pointes or with long QT syndrome.

4. Opioid antagonist for use according to claim 1, wherein the arrhythmia is induced by the administration of buprenorphine or a pharmaceutically acceptable salt thereof.

5. Opioid antagonist for use according to claim 1, wherein the opioid antagonist is naltrexone or a pharmaceutically acceptable salt thereof.

6. Opioid antagonist for use according to claim 1, wherein the opioid antagonist is for administration by a route selected from the group consisting of oral, transdermal, sublingual, buccal, gingival, rectal, subcutaneous, intramuscular, intravenous and parenteral routes.

7. Opioid antagonist for use according to any of claims 1 to 6, wherein the patient is a human subject.

## Patentansprüche

1. Opioid-Antagonist zur Verwendung in einem Verfahren zum Vorbeugen oder Behandeln von Arrhythmie bei einem Patienten durch Verringern des QTcI bei dem Patienten auf unter 10 ms, wobei Arrhythmie durch die Verabreichung eines Opioidanalgetikums induziert ist, wobei das Verfahren Verabreichen einem Patienten, der dies benötigt, einer wirksamen Menge des Opioid-Antagonisten gleichzeitig mit dem Opioid-Analgetikum umfasst, um der Arrhythmie vorzubeugen oder diese zu behandeln, wobei das Opioid-Analgetikum ausgewählt ist aus der Gruppe, bestehend aus Oxycodon, Morphin, Codein, Oxymorphon, Fentanyl, Hydrocodon, Hydromorphon, Tramadol, Buprenorphin, Methadon und pharmazeutisch annehmbaren Salzen davon, und wobei der Opioid-Antagonist ausgewählt ist aus der Gruppe, bestehend aus Naltrexon, Naloxon, Nalmefen, Cyclazacin, Levallorphan und pharmazeutisch annehmbaren Salzen davon.

2. Opioid-Antagonist zur Verwendung nach Anspruch 1, wobei die Arrhythmie ein verlängertes QT-Intervall ist.

3. Opioid-Antagonist zur Verwendung nach Anspruch 2, wobei bei dem Patienten ventrikuläre Tachyarrhythmie oder Torsade de pointes oder das Long-QT-Syndrom diagnostiziert wurde.

4. Opioid-Antagonist zur Verwendung nach Anspruch 1, wobei die Arrhythmie durch die Verabreichung von Buprenorphin oder einem pharmazeutisch annehmbaren Salz davon verursacht ist.

5. Opioid-Antagonist zur Verwendung nach Anspruch 1, wobei der Opioid-Antagonist Naltrexon oder ein pharmazeutisch annehmbares Salz davon ist.

6. Opioid-Antagonist zur Verwendung nach Anspruch 1, wobei der Opioid-Antagonist auf einem Weg zu verabreichen ist, gewählt aus der Gruppe, bestehend aus dem oralen, transdermalen, sublingualen, bukkalen, gingivalen, rektalen, subkutanen, intramuskulären, intravenösen und parenteralen Wege.

7. Opioid-Antagonist zur Verwendung nach einem der Ansprüche 1 bis 6, wobei der Patient ein Mensch ist.

## Revendications

1. Antagoniste des opioïdes pour son utilisation dans un procédé de prévention ou de traitement d'une arythmie chez un patient par la diminution du QTcI chez ledit patient à moins de 10 ms, dans lequel l'arythmie est induite par l'administration d'un analgésique opioïde, le procédé comprenant l'administration à un patient en ayant besoin d'une quantité efficace de l'antagoniste des opioïdes en même temps que l'analgésique opioïde pour prévenir ou traiter l'arythmie, dans lequel l'analgésique opioïde est sélectionné dans le groupe constitué de l'oxycodone, de la morphine, de la codéine, de l'oxymorphone, du fentanyl, de l'hydrocodone, de l'hydromorphone, du tramadol, de la buprénorphine, de la méthadone, et des sels pharmaceutiquement acceptables de ceux-ci, et dans lequel l'antagoniste opioïde est sélectionné dans le groupe constitué de la naltrexone, de la naloxone, du nalméfène, de la cyclazacine, du lévallorphane, et des sels pharmaceutiquement acceptables de ceux-ci.

2. Antagoniste des opioïdes pour son utilisation selon la revendication 1, dans lequel l'arythmie est un intervalle QT prolongé.

3. Antagoniste des opioïdes pour son utilisation selon la revendication 2, dans lequel le patient est diagnostiqué avec une tachyarythmie ventriculaire ou avec des torsades de pointes ou avec un syndrome du QT long.

4. Antagoniste des opioïdes pour son utilisation selon la revendication 1, dans lequel l'arythmie est induite par l'administration de buprénorphine ou d'un sel pharmaceutiquement acceptable de celle-ci.

5. Antagoniste des opioïdes pour son utilisation selon la revendication 1, dans lequel l'antagoniste opioïde est la naltrexone ou un sel pharmaceutiquement acceptable de celle-ci.

6. Antagoniste des opioïdes pour son utilisation selon la revendication 1, dans lequel l'antagoniste opioïde est pour une administration par une voie sélectionnée dans le groupe constitué des voies orale, transdermique, sublinguale, buccale, gingivale, rectale, sous-cutanée, intramusculaire, intraveineuse et parentérale.

7. Antagoniste des opioïdes pour son utilisation selon une quelconque des revendications 1 à 6, dans lequel le patient est un sujet humain.
